(19) Europäisches Patentamt
European Patent Office
Office européen des brevets

(11) EP 2 187 850 B1

(12) EUROPEAN PATENT SPECIFICATION

(45) Date of publication and mention
of the grant of the patent:
**16.02.2011 Bulletin 2011/07**

(51) Int Cl.:
***A61F 9/00*** (2006.01)

(21) Application number: **08838112.4**

(22) Date of filing: **01.10.2008**

(86) International application number:
**PCT/US2008/078457**

(87) International publication number:
**WO 2009/048777 (16.04.2009 Gazette 2009/16)**

(54) **THERMAL COEFFICIENT DRIVEN DRUG PELLET SIZE FOR OPHTHALMIC INJECTION**

WÄRMEKOEFFIZIENTENGESTEUERTE GRÖSSE VON MEDIKAMENTENPILLEN ZUR INJEKTION IN DIE AUGEN

GROSSEUR DE GRANULES DE MÉDICAMENT SOUMIS À UN COEFFICIENT THERMIQUE POUR INJECTION OPHTALMIQUE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **09.10.2007 US 978456 P**

(43) Date of publication of application:
**26.05.2010 Bulletin 2010/21**

(73) Proprietor: **Alcon Research, Ltd.**
**Fort Worth TX 76134 (US)**

(72) Inventors:
• **SANCHEZ, JR., Robert**
**Oceanside, California 92054 (US)**
• **DOS SANTOS, Cesario**
**Aliso Viejo, California 92656 (US)**

(74) Representative: **Hanna, Peter William Derek et al**
**Hanna Moore & Curley**
**13 Lower Lad Lane**
**Dublin 2 (IE)**

(56) References cited:
**WO-A2-2007/024369**

## Description

## BACKGROUND OF THE INVENTION

The present invention relates to a method of delivering medication into the eye, and more particularly to delivering a phase transition or reverse gelation compound / drug mixture in an intraocular injection.

Several diseases and conditions of the posterior segment of the eye threaten vision. Age related macular degeneration (ARMD), choroidal neovascularization (CNV), retinopathies (e.g., diabetic retinopathy, vitreoretinopathy), retinitis (e.g., cytomegalovirus (CMV) retinitis), uveitis, macular edema, glaucoma, and neuropathies are several examples.

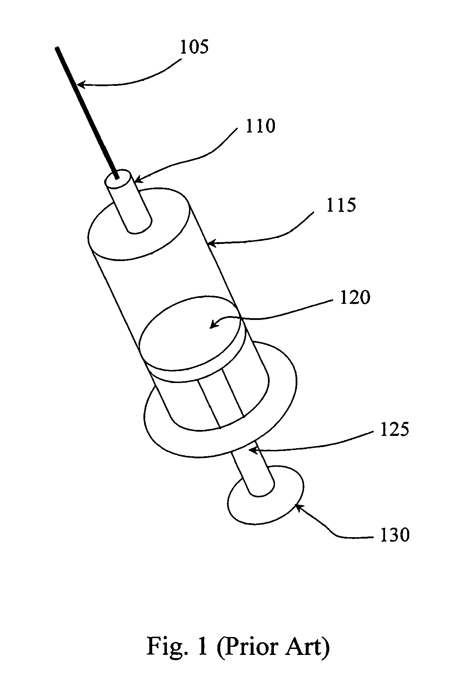

These, and other diseases, can be treated by injecting a drug into the eye. Such injections are typically manually performed using a conventional syringe and needle. Figure 1 is a perspective view of a prior art syringe used to inject drugs into the eye. In Figure 1, the syringe includes a needle 105, a luer hub 110, a chamber 115, a plunger 120, a plunger shaft 125, and a thumb rest 130. As is commonly known, the drug to be injected is located in chamber 115. Pushing on the thumb rest 130 causes the plunger 120 to expel the drug through needle 105.

In using such a syringe, the surgeon is required to pierce the eye tissue with the needle, hold the syringe steady, and actuate the syringe plunger (with or without the help of a nurse) to inject the fluid into the eye. Fluid flow rates are uncontrolled. Reading the vernier is subject to parallax error which affects the precision and accuracy of the injected volume. Tissue damage may occur due to an "unsteady" injection. Reflux of the drug may also occur when the needle is removed from the eye.

An effort has been made to control the delivery of small amounts of liquids. A commercially available fluid dispenser is the ULTRA™ positive displacement dispenser available from EFD Inc. of Providence, Rhode Island. The ULTRA dispenser is typically used in the dispensing of small volumes of industrial adhesives. It utilizes a conventional syringe and a custom dispensing tip. The syringe plunger is actuated using an electrical stepper motor and an actuating fluid. With this type of dispenser, the volumes delivered are highly dependent on fluid viscosity, surface tension, and the specific dispensing tip. Parker Hannifin Corporation of Cleveland, Ohio distributes a small volume liquid dispenser for drug discovery applications made by Aurora Instruments LLC of San Diego, California. The Parker/Aurora dispenser utilizes a piezo-electric dispensing mechanism. While precise, this dispenser is expensive and requires an electrical signal to be delivered to the dispensing mechanism.

U.S. Patent No. 6,290,690 discloses an ophthalmic system for injecting a viscous fluid (e.g. silicone oil) into the eye while simultaneously aspirating a second viscous fluid (e.g. perflourocarbon liquid) from the eye in a fluid/fluid exchange during surgery to repair a retinal detachment or tear. The system includes a conventional syringe with a plunger. One end of the syringe is fluidly coupled to a source of pneumatic pressure that provides a constant pneumatic pressure to actuate the plunger. The other end of the syringe is fluidly coupled to an infusion cannula via tubing to deliver the viscous fluid to be injected.

Document WO-A-2007/024369 discloses a method of preparing an injection device for injecting a mixture into an eye comprising the step of providing the mixture in a dispensing chamber, bringing the dispensing chamber housing and mixture to a temperature range, other than near room temperature, at which the mixture expands and is in a more liquid state.

It would be desirable to effectively inject a drug into the eye. When a drug is to be injected into the eye, it is desirable to minimize the number of injections. A spherical bolus of drug can erode over time at a known rate. Depositing such a spherical bolus in the eye can prolong the time between injections. It would be desirable to control the temperature and rate at which a drug is delivered into the eye so as to regulate the time period over which the drug is delivered to the retina.

## SUMMARY OF THE INVENTION

The present invention is a method of preparing an injection device for injecting a mixture into an eye according to claim 1.

It is to be understood that both the foregoing general description and the following detailed description are exemplary and explanatory only and are intended to provide further explanation of the invention as claimed. The following description, as well as the practice of the invention, set forth and suggest additional advantages and purposes of the invention.

## BRIEF DESCRIPTION OF THE DRAWINGS

The accompanying drawings, which are incorporated in and constitute a part of this specification, illustrate several embodiments of the invention and together with the description, serve to explain the principles of the invention.

Figure 1 is a perspective view of a prior art syringe.

Figure 2 is a view of an ophthalmic medical device including a disposable tip segment and a limited reuse assembly.

Figure 3 is an embodiment of a limited reuse assembly.

Figure 4 is a cross section view of a disposable tip segment for an ophthalmic hand piece.

Figure 5 is cross section view of a disposable tip segment and a limited reuse assembly.

Figures 6A, 6B, 6C are cross section views of a dispensing chamber housing including a drug suspended in a phase transition compound.

Figure 7 is a view of an injection needle inserted into an eye.

Figure 8 is a cross section view of various bolus shapes for injection into the eye.

Figure 9 is a method of injecting a rate and temperature dependent substance into the eye according to the principles of the present invention.

Figure 10 is a method of injecting a rate and temperature dependent substance into the eye according to the principles of the present invention.

## DETAILED DESCRIPTION OF THE PREFERRED EMBODIMENTS

Reference is now made in detail to the exemplary embodiments of the invention, examples of which are illustrated in the accompanying drawings. Wherever possible, the same reference numbers are used throughout the drawings to refer to the same or like parts.

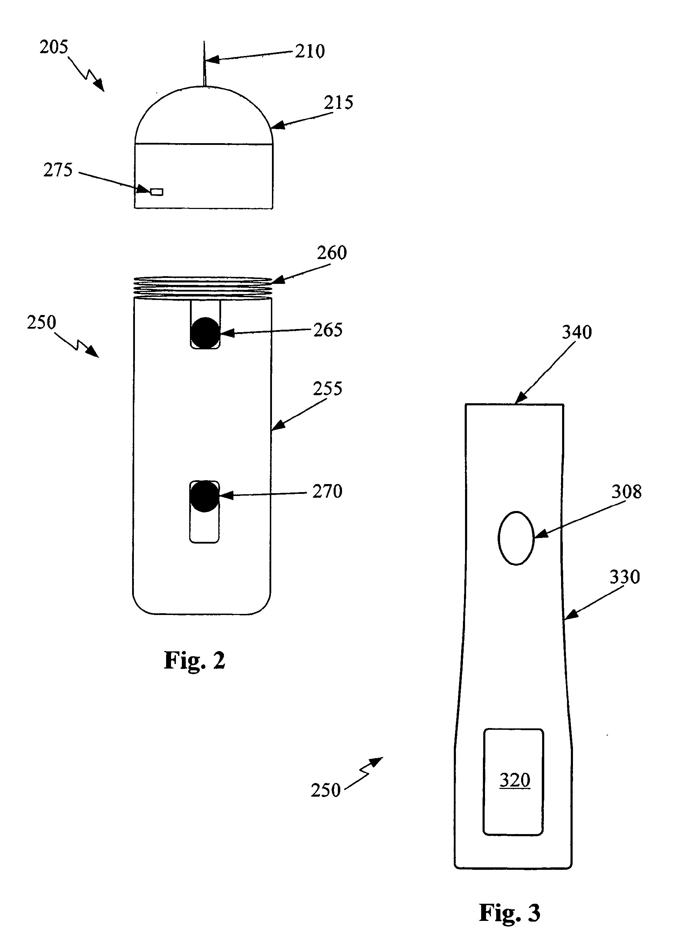

Figure 2 is one view of an ophthalmic medical device including a disposable tip segment and a limited reuse assembly. In Figure 2, the medical device includes a tip segment 205 and a limited reuse assembly 250. The tip segment 205 includes a needle 210, a housing 215, and an optional light 275. The limited reuse assembly 250 includes a housing 255, a switch 270, a lock mechanism 265, and a threaded portion 260.

Tip segment 205 is capable of being connected to and removed from limited reuse assembly 250. In this embodiment, tip segment 205 has a threaded portion on an interior surface of housing 215 that screws onto the threaded portion 260 of limited reuse assembly 250. In addition, lock mechanism 265 secures tip segment 215 to limited reuse assembly 250. Lock mechanism 265 may be in the form of a button, a sliding switch, or a cantilevered mechanism. Other mechanisms for connecting tip segment 205 to limited reuse assembly 250, such as those involving structural features that mate with each other, are commonly known in the art and may also be employed.

Needle 210 is adapted to deliver a substance, such as a drug, into an eye. Needle 210 may be of any commonly known configuration. Preferably, needle 210 is designed such that its thermal characteristics are conducive to the particular drug delivery application. For example, when a heated drug is to be delivered, needle 210 may be relatively short (several millimeters) in length to facilitate proper delivery of the drug.

Switch 270 is adapted to provide an input to the system. For example, switch 270 may be used to activate the system or to turn on a heater. Other switches, buttons, or user-directed control inputs are commonly known and may be employed with limited reuse assembly 250 and / or tip segment 205.

Optional light 275 is illuminated when tip segment 205 is ready to be used. Optional light 275 may protrude from housing 215, or it may be contained within housing 215, in which case, optional light 275 may be seen through a clear portion of housing 215. In other embodiments, optional light 275 may be replaced by an indicator, such as a liquid crystal display, segmented display, or other device that indicates a status or condition of disposable tip segment 205. For example, optional light 275 may also pulse on and off to indicate other states, such as, but not limited to a system error, fully charged battery, insufficiently charged battery or faulty connection between the tip segment 205 and limited use assembly 250. While shown on tip segment 205, optional light 275 or other indicator may be located on limited reuse assembly 250.

Figure 3 is another embodiment of a limited reuse assembly. Limited reuse assembly 250 includes a button 308, a display 320, and a housing 330. Disposable tip segment 205 attaches to end 340 of limited reuse assembly 250. Button 308 is actuated to provide an input to the system. As with switch 270, button 308 may activate a heater or other temperature control device or initiate actuation of a plunger. Display 320 is a liquid crystal display, segmented display, or other device that indicates a status or condition of disposable tip segment 205 or limited reuse assembly 250.

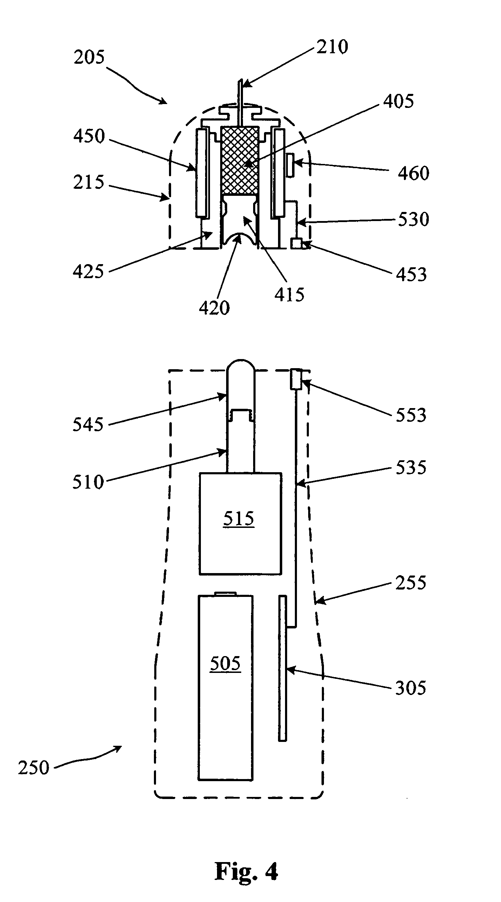

Figure 4 is cross section view of a disposable tip segment and a limited reuse assembly. Figure 4 shows how tip segment 205 interfaces with limited reuse assembly 250. In the embodiment of Figure 4, tip segment 205 includes plunger interface 420, plunger 415, dispensing chamber housing 425, tip segment housing 215, temperature control device 450, thermal sensor 460, needle 210, dispensing chamber 405, interface 530, and tip interface connector 453. Limited reuse assembly 250 includes mechanical linkage interface 545, actuator shaft 510, actuator 515, power source 505, controller 305, limited reuse assembly housing 255, interface 535, and limited reuse assembly interface connector 553.

In tip segment 205, plunger interface 420 is located on one end of plunger 415. The other end of plunger 415 forms one end of dispensing chamber 405. Plunger 415 is adapted to slide within dispensing chamber 405. The outer surface of plunger 415 is fluidly sealed to the inner surface of dispensing chamber housing 425. Dispensing chamber housing 425 surrounds the dispensing chamber 405. Typically, dispensing chamber housing 425 has a cylindrical shape. As such, dispensing chamber 405 also has a cylindrical shape.

Needle 210 is fluidly coupled to dispensing chamber 405. In such a case, a substance contained in dispensing chamber 405 can pass through needle 210 and into an eye. Temperature control device 450 at least partially surrounds dispensing chamber housing 425. In this case, temperature control device 450 is adapted to heat and/or cool dispensing chamber housing 425 and any substance contained in dispensing chamber 405. Interface 530 connects temperature control device 450 with tip interface connector 453.

Optional thermal sensor 460 provides temperature information to assist in controlling the operation of temperature control device 450. Thermal sensor 460 may be located near dispensing chamber housing 425 and measure a temperature near dispensing chamber housing 425 or may be located in thermal contact with dispensing chamber housing 425, in which case it measures a temperature of dispensing chamber housing 425. Thermal sensor 460 may be any of a number of different devices that can provide temperature information. For example, thermal sensor 460 may be a thermocouple or a resistive device whose resistance varies with temperature. Thermal sensor is also electrically coupled to interface 530 or other similar interface.

The components of tip segment 205, including dispensing chamber housing 425, temperature control device 450, and plunger 415 are at least partially enclosed by tip segment housing 215. Plunger 415 is sealed to the interior surface of dispensing chamber housing 425. This seal prevents contamination of any substance contained in dispensing chamber 405. For medical purposes, such a seal is desirable. This seal can be located at any point on plunger 415 or dispensing chamber housing 425.

In limited reuse assembly 250, power source 505 provides power to actuator 515. An interface (not shown) between power source 505 and actuator 515 serves as a conduit for providing power to actuator 515. Actuator 515 is connected to actuator shaft 510. When actuator 515 is a stepper motor, actuator shaft 510 is integral with actuator 515. Mechanical linkage interface 545 is connected to actuator shaft 510. In this configuration, as actuator 515 moves actuator shaft 510 upward toward needle 210, mechanical linkage interface 545 also moves upward toward needle 210. Mechanical linkage interface 545 and actuator shaft 510 are a single component. In other words, a shaft connected to actuator 515 includes both actuator shaft 510 and mechanical linkage interface 545 as a single assembly.

In limited reuse assembly 250, power source 505 is typically a rechargeable battery, such as a lithium ion battery, although other types of batteries may be employed. In addition, any other type of power cell is appropriate for power source 505. Power source 505 provides current to dispensing chamber housing 425 to heat it and change its shape. Optionally, power source 505 can be removed from housing 255 through a door or other similar feature (not shown).

Controller 305 is connected via interface 535 to limited reuse assembly interface connecter 553. Limited reuse assembly interface connecter 553 is located on a top surface of limited reuse assembly housing 255 adjacent to mechanical linkage interface 545. In this manner, both limited reuse assembly interface connector 553 and mechanical linkage interface 545 are adapted to be connected with tip interface connector 453 and plunger interface 420, respectively.

Controller 305 and actuator 515 are connected by an interface (not shown). This interface (not shown) allows controller 305 to control the operation of actuator 515. In addition, an interface between power source 505 and controller 305 allows controller 305 to control operation of power source 505. In such a case, controller 305 may control the charging and the discharging of power source 505 when power source 505 is a rechargeable battery.

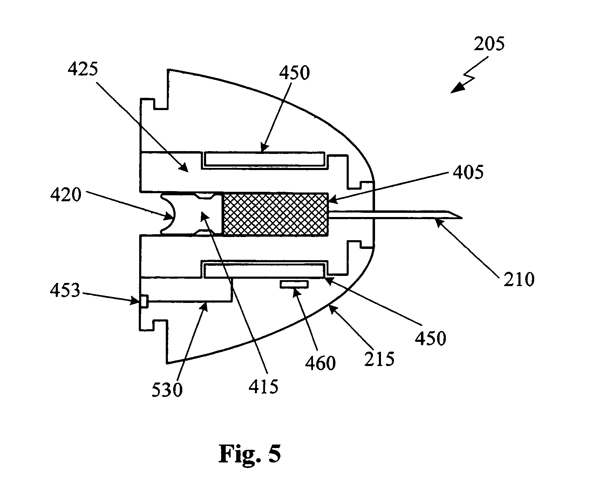

Controller 305 is typically an integrated circuit with power, input, and output pins capable of performing logic functions. In various embodiments, controller 305 is a targeted device controller. In such a case, controller 305 performs specific control functions targeted to a specific device or component, such as a temperature control device or a power supply. For example, a temperature control device controller has the basic functionality to control a temperature control device. In other embodiments, controller 305 is a microprocessor. In such a case, controller 305 is programmable so that it can function to control more than one component of the device. In other cases, controller 305 is not a programmable microprocessor, but instead is a special purpose controller configured to control different components that perform different functions. While depicted as one component in Figure 5, controller 305 may be made of many different components or integrated circuits.

Tip segment 205 is adapted to mate with or attach to limited reuse assembly 250. In the embodiment of Figure 4, plunger interface 420 located on a bottom surface of plunger 415 is adapted to mate with mechanical linkage interface 545 located near a top surface of limited reuse assembly housing 255. In addition, tip interface connector 453 is adapted to connect with limited reuse assembly interface connector 553. When tip segment 205 is connected to limited reuse assembly 250 in this manner, actuator 515 and actuator shaft 510 are adapted to drive plunger 415 upward toward needle 210. In addition, an interface is formed between controller 305 and temperature control device 450. A signal can pass from controller 305 to temperature control device 450 through interface 535, limited reuse assembly interface connector 553, tip interface connector 453, and interface 530.

In operation, when tip segment 205 is connected to limited reuse assembly 250, controller 305 controls the operation of actuator 515. When actuator 515 is actuated, actuator shaft 510 is moved upward toward needle 210. In turn, mechanical linkage interface 545, which is mated with plunger interface 420, moves plunger 415 upward toward needle 210. A substance located in dispensing chamber 405 is then expelled through needle 210.

In addition, controller 305 controls the operation of temperature control device 450. Temperature control device 450 is adapted to heat and/or cool dispensing chamber housing 425 and its contents. Since dispensing chamber housing 425 is at least partially thermally conductive, heating or cooling dispensing chamber housing 425 heats or cools a substance located in dispensing chamber 405. Temperature information can be transferred from thermal sensor 460 through interface 530, tip interface connector 453, limited reuse assembly interface connector 553, and interface 535 back to controller 305. This temperature information can be used to control the operation of temperature control device 450. When temperature control device 450 is a heater, controller 305 controls the amount of current that is sent to temperature control device 450. The more current sent to temperature control device 450, the hotter it gets. In such a manner, controller 305 can use a feed back loop utilizing information from thermal sensor 460 to control the operation of temperature control device 450. Any suitable type of control algorithm, such as a proportional integral derivative (PID) algorithm, can be used to control the operation of temperature control device 450.

A substance to be delivered into an eye, typically a drug suspended in a phase transition compound, is located in dispensing chamber 405. In this manner, the drug and phase transition compound are contacted by the inner surface of dispensing chamber housing 425. The phase transition compound is in a solid or semi-solid state at lower temperatures and in a more liquid state at higher temperatures. Such a compound can be heated by the application of current to temperature control device 450 to a more liquid state and injected into the eye where it forms a bolus that erodes over time.

Likewise, a reverse gelation compound may be used. A reverse gelation compound is in a solid or semi-solid state at higher temperatures and in a more liquid state at lower temperatures. Such a compound can be cooled by temperature control device 450 to a more liquid state and injected into the eye where it forms a bolus that erodes over time. As such, temperature control device 450 may be a device that heats a substance in dispensing chamber 405 or a device that cools a substance in dispensing chamber 405 (or a combination of both). After being delivered into the eye, a phase transition compound or reverse gelation compound erodes over time providing a quantity of drug over an extended period of time. Using a phase transition compound or reverse gelation compound provides better drug dosage with fewer injections.

In one embodiment, the substance located in dispensing chamber 405 is a drug that is preloaded into the dispensing chamber. In such a case, tip segment 205 is appropriate as a single use consumable product. Such a disposable product can be assembled at a factory with a dosage of a drug installed.

While shown as a two-piece device, the injection system of Figure 4 may be a single piece device. In such a case, the tip segment is integrated into the limited reuse assembly to form a single medical device.

Figure 5 is a cross section view of a tip segment for an ophthalmic medical device. In Figure 5, tip segment 205 includes dispensing chamber housing 425, tip segment housing 215, thermal sensor 460, needle 210, dispensing chamber 405, plunger 415, plunger interface 420, temperature control device 450, interface 530, and tip interface connector 453.

In the embodiment of Figure 5, temperature control device 450 is activated to bring a substance in dispensing chamber 405 to within a proper temperature range. Thermal sensor 460 provides temperature information to controller 305 (not shown) to control temperature control device 450. After the substance has reached the proper temperature range, plunger 415 is actuated to deliver the substance through needle 210 and into an eye. Plunger 415 is extended and includes an integral shaft as shown.

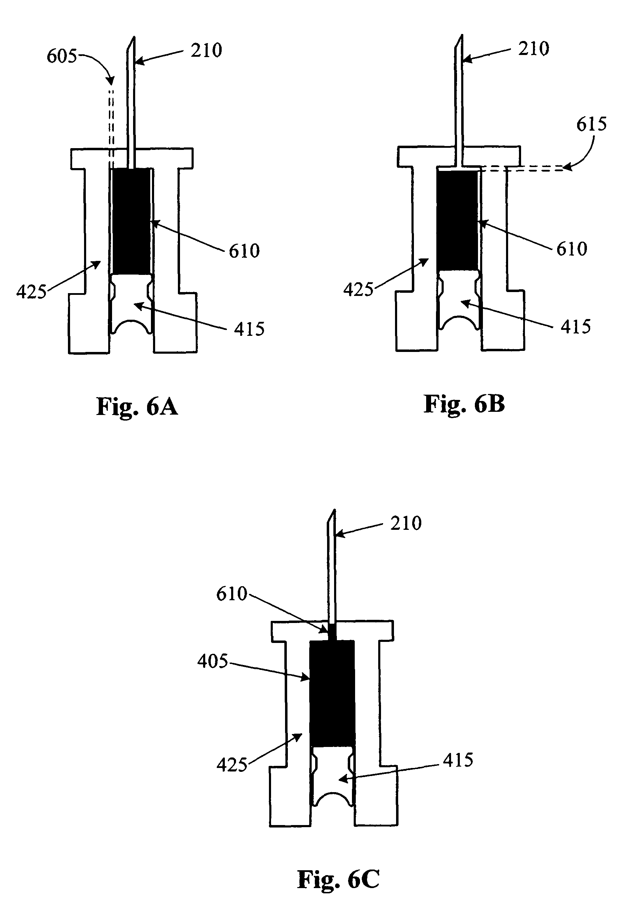

Figures 6A, 6B, and 6C are cross section views of a dispensing chamber housing including a drug suspended in a phase transition compound. In figure 6A, dispensing chamber housing 425 holds a pellet 610 of a drug suspended in a phase transition compound. An air gap 605 exists between the pellet 610 and the interior surface of dispensing chamber housing 425. This air gap 605 may be uniform or nonuniform in nature. However, the volume of air gap 605 is a calculated quantity as explained below. Needle 210 and plunger 415 are also depicted. In Figure 6B, the air gap 615 exists between the top interior surface of dispensing chamber housing 425 and pellet 610. Accordingly, the location of the air gap (605 or 615 as the case may be) is not as important as the volume of the air gap provided the air gap does not exist between plunger 415 and pellet 610. A significant amount of air entrapped between plunger 415 and pellet 610 can lead to the drug bubbling out of needle 210 as the temperature of the pellet is altered (and the air expands and force the drug through needle 210) and the pellet liquefies.

Figure 6C shows the pellet 610 after it has reached the proper injection temperature. When the pellet 610 is a drug suspended in a phase transition compound, the pellet is heated as dispensing chamber housing 425 is heated. Needle 210 is also heated. The pellet 610 expands as it is heated. As the pellet expands, the air in the air gap (605 or 615 as the case may be) escapes through needle 210. The pellet 610 expands to substantially fill the dispensing chamber or volume enclosed by dispensing chamber housing 425 and plunger 415. A small amount of the pellet may also expand into needle 210. However, it is important to keep air in the protruding portion of needle 210 to prevent the drug / phase transition compound mixture from solidifying in needle 210 and blocking it during injection. Since needle 210 is cooler than dispensing chamber housing 425 and pellet 610, if any significant amount of the drug / phase transition compound mixture enters the protruding portion of needle 210, it rapidly cools and solidifies, blocking the needle 210. Accordingly, applicants have made the discovery that keeping air in needle 210 and injecting that air into the eye along with the pellet 610 is beneficial for a controlled injection.

When the pellet 610 and the dispensing chamber bounded by the dispensing chamber housing 425 are both cylindrical, the air gap is calculated by using the formula for the volume of a cylinder. The volume of the dispensing chamber is denoted by $V_{DC}$, the volume of the pellet at a first temperature is denoted by $V_{P1}$, and the volume of the pellet at a second temperature is denoted by $V_{P2}$:

$$V_{DC} = \pi R_{DC}^2 \cdot H_{DC}$$

$$V_{P1} = \pi R_{P1}^2 \cdot H_{P1}$$

$$V_{P2} = \pi R_{P2}^2 \cdot H_{P2}$$

where $R_{DC}$ and $H_{DC}$ are the radius and height, respectively, of the dispensing chamber, $R_{P1}$ and $H_{P1}$ are the radius and height, respectively, of the cylindrical pellet at a first temperature, and $R_{P2}$ and $H_{P2}$ are the radius and height, respectively, of the cylindrical pellet at a second temperature. In this example, the volume of the dispensing chamber is known and does not change. Since the pellet is made of a phase transition compound, its volume changes as a function of temperature. When the pellet is made of a phase transition compound / drug mixture (Precirol / pharmaceutical), the first temperature is 20 to 23 degrees Celsius, and the second temperature is 75 degrees Celsius, it was found that $V_{T2} = 1.2 \cdot V_{T1}$ (where 1.2 is an example value of a thermal coefficient of expansion at 75 degrees Celsius from 23 degrees Celsius). In other words, the volume of the pellet increases by twenty percent when it is heated to 75 degrees Celsius from room temperature. The air gap is then calculated by taking the difference between $V_{T2}$ and $V_{T1}$ (i.e. $V_{T2} - V_{T1}$ = volume of air gap). This volume of air gap can then be maintained in the dispensing chamber by forming a pellet with the volume, $V_{T1}$. A pellet with this volume (and any shape) can then be placed in the dispensing chamber on top of the plunger.

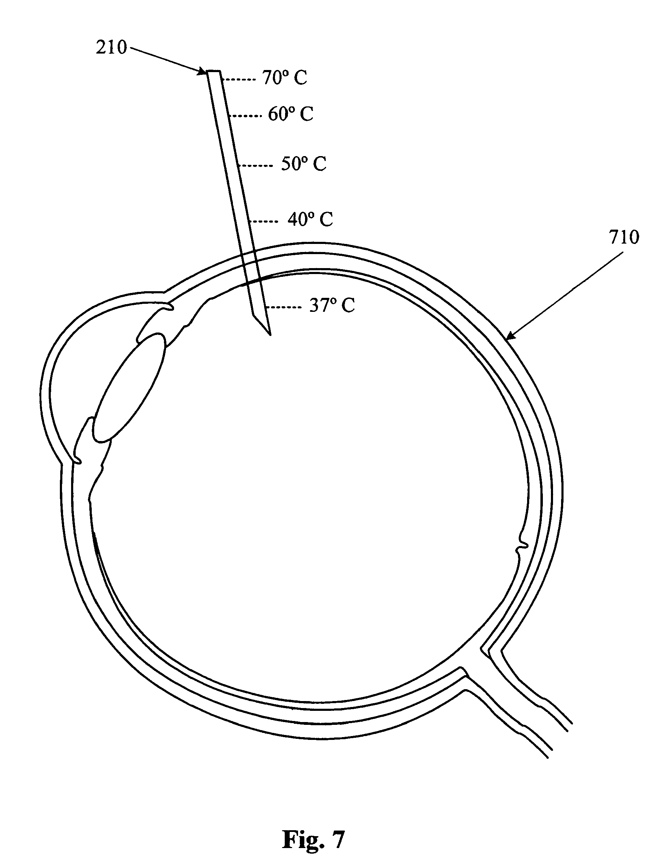

Figure 7 is a view of an injection needle inserted into an eye. In this case, the dispensing chamber housing and pellet are heated to 75 degrees Celsius. The measured temperature of the needle during an injection into the eye is shown. Needle 210 is inserted into the posterior segment of eye 710. Since eye 710 has a very large thermal mass compared to needle 710, the tip of needle 710 cools very quickly (almost instantaneously) to the temperature of the eye. A temperature gradient develops between the needle 210 and the eye 710. The end of the needle closest to the dispensing chamber housing (and heater) is hotter than the end of the needle 210 in the eye. Because of this difference in temperature, it is important to keep an air gap in needle 210 prior to an injection to prevent the drug / phase transition compound mixture from cooling. This leads to the counterintuitive reasoning of keeping air in a needle that is used for an injection. Typically, all air is evacuated out of a needle before an injection. However, a small amount of air injected into the eye is not harmful, and the presence of air in the needle allows for the injection to take place.

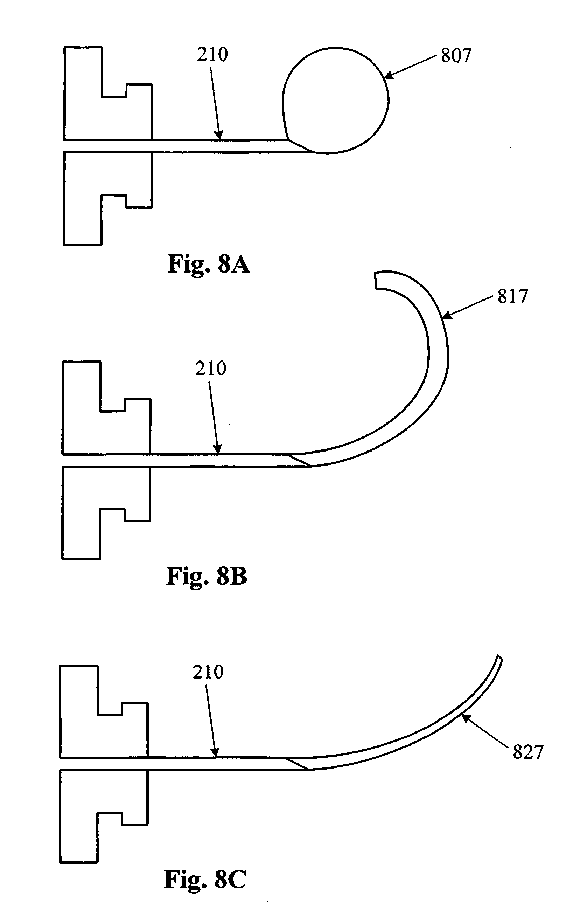

Figure 8 is a cross section view of various bolus shapes for injection into the eye. Figure 8A depicts a bolus 807 of a preferred spherical or nearly spherical shape. The near spherical shape of bolus 807, when deposited into the eye, allows the drug contained in bolus 807 to erode over time with a known dosage of drug being delivered. Figure 8B depicts a cylindrical shape 817 that results if the injection speed is too slow. Figure 8C depicts an elongated cylindrical shape that results if the injection speed is too fast.

The rate at which the injection takes place (for a given substance at a given temperature) determines the resulting shape of the injection. The Applicants have experimented with substances discussed in U.S. Patent Application No. 11/695,990 filed on April 3, 2007. These substances are lipophilic compounds with temperature profiles suitable for intraocular use. Some of these compounds remain in a solid or semi-solid state near 37 degrees Celsius (the temperature of the human body), and can be heated to a more liquid state above 37 degrees Celsius. It has been found that heating a phase transition compound, such as these, to a temperature of about 75 degrees Celsius keeps it in a liquid or near liquid state so that it can be injected into an eye. The compound then cools to 37 degrees Celsius where it remains in a solid or semi-solid state. Such injections typically have a volume of a few to tens of microliters.

For example, when using a Precirol / pharmaceutical mixture (a phase transition compound / drug mixture), it was found that heating the mixture to 75 degrees Celsius keeps it in a liquid state. It can then be injected into the eye to form a bolus. A rapid injection rate (rate greater than about 14 in./min.) results in the elongated cylindrical shape of Figure 8C. This shape results because of convective and conductive cooling associated with the rapid injection rate. The mixture exits the needle at such a rapid velocity that it does not form a cylindrical bolus. A slow injection rate (rate about 10-12 in./min.) results in the cylindrical shape of Figure 8B. This shape results because of convective cooling associated with the slow injection rate. The mixture exits the needle so slowly that it cools and solidifies forming a cylinder. An injection rate of about 8-10 in./min. results in the bolus of Figure 8A. This injection range was found to be optimal for creating a spherical or near spherical bolus. Variations in this range can produce variations in the bolus shape, making it less spherical. Additionally, the different shapes (cylindrical, spherical, or other) each have different surface areas that correspond to different drug release rates because the rate of erosion of the shape in the eye depends on its surface area.

These experiments were conducted using a 27 gauge needle, which is preferable because of its small size and because of the small dosages delivered (on the order of microliters). Needles with other gauges can also be used. However, it is preferable to use small gauge needles that create a self-sealing injection wound. Typically, needles smaller than 25 gauge are preferred.

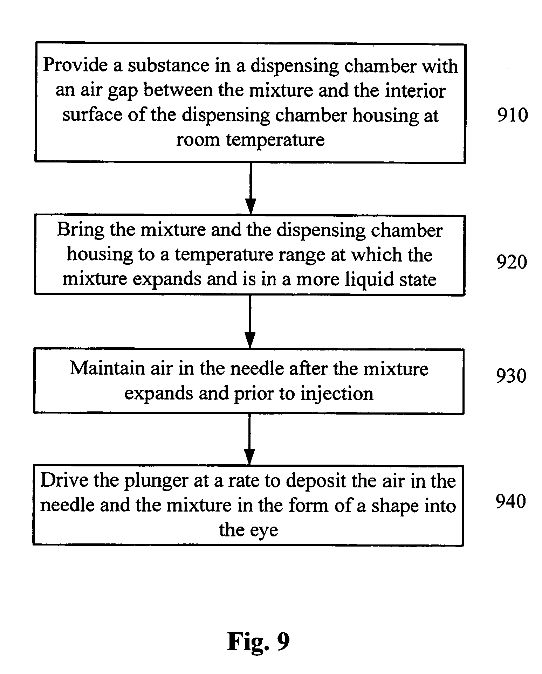

Figure 9 is a method of injecting a rate and temperature dependent substance into the eye according to the principles of the present invention. In 910, a substance is provided in a dispensing chamber with an air gap between the substance and the interior surface of the dispensing chamber housing at room temperature. In 920, the substance and the dispensing chamber housing are brought to a temperature range at which the substance expands and is in a more liquid state. The air gap between the interior surface of the dispensing chamber housing and the substance can be calculated as described above. The air gap is based on the thermal expansion characteristics of the substance. As the temperature of the substance changes and it expands, air is expelled through the needle prior to injection. However, in 930, air is maintained in the needle after the substance expands and before the substance is injected into the eye. In 940, the plunger is driven at a rate to deposit the air in the needle and the substance in the form of a shape into the eye.

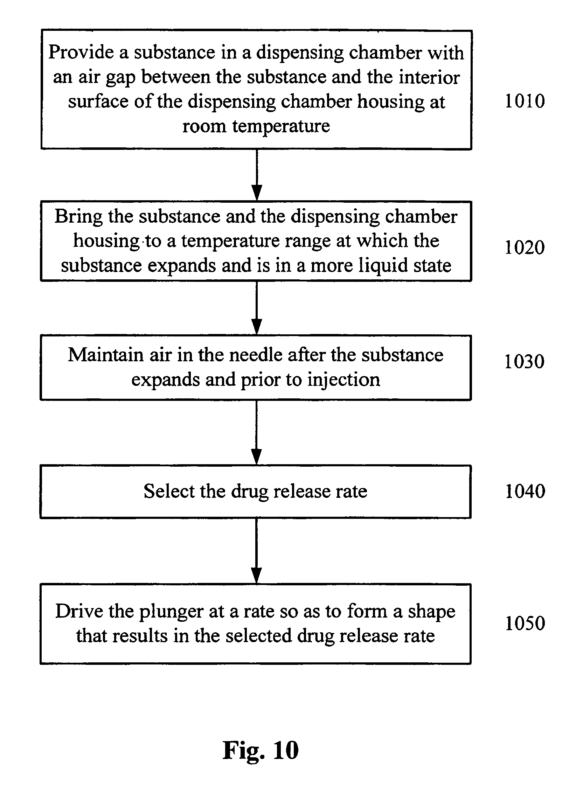

Figure 10 is a method of injecting a rate and temperature dependent substance into the eye according to the principles of the present invention. In 1010, a substance is provided in a dispensing chamber with an air gap between the substance and the interior surface of the dispensing chamber housing at room temperature. In 1020, the substance and the dispensing chamber housing are brought to a temperature range at which the substance expands and is in a more liquid state. The air gap between the interior surface of the dispensing chamber housing and the substance can be calculated as described above. The air gap is based on the thermal expansion characteristics of the substance. As the temperature of the substance changes and it expands, air is expelled through the needle prior to injection. However, in 1030, air is maintained in the needle after the substance expands and before the substance is injected into the eye. In 1040, a drug release rate is selected. This drug release rate may be selected from a range of drug release rates. In 1050, the plunger is driven at a rate so as to form a shape in the eye that results in the selected drug release rate.

As previously explained, the surface area of the shape of the phase transition compound / drug mixture deposited in the eye determines the release rate of the drug. Since the erosion of the mixture in the eye is dependent on its surface area, the shape of that mixture (spherical, cylindrical, or some other shape) influences the rate of erosion and consequent drug release rate. In most cases, it is desirable to maximize the duration between injections (and minimize the surface area) by depositing a near-spherical bolus in the eye. However, it may be desirable to increase drug delivery rates by depositing other shapes with greater surface area (such as cylindrical shapes). Drug delivery rates are also dependent on the type of substance and concentration of drug - both of which can be selected to provide pellets suitable for varying dosages based on varying the shape of the injection.

From the above, it may be appreciated that the present invention provides an improved system and method for delivering precise volumes of a substance into the eye. The present invention provides method of injecting a substance into the eye. A substance / drug mixture may be heated or cooled (as the case may be) to transform it into a more liquid state that is suitable for injection into the eye. The presence of an air gap in the dispensing chamber before the temperature of the mixture is altered, and air in the needle after it is altered, produce suitable injection results. The present invention is illustrated herein by example, and various modifications may be made by a person of ordinary skill in the art.

Other embodiments of the invention will be apparent to those skilled in the art from consideration of the specification and practice of the invention disclosed herein. It is intended that the specification and examples be considered as exemplary only, with a true scope and of the invention being indicated by the following claims.

## Claims

1. A method of preparing an injection device (250) for injecting a mixture into an eye comprising:

   providing (910) the mixture in a dispensing chamber (405) with an air gap located between the mixture and an interior surface of a dispensing chamber housing (425), when the mixture and dispensing chamber housing are near room temperature;
   bringing (920) the dispensing chamber housing and mixture to a temperature range, other than near room temperature, at which the mixture expands and is in a more liquid state;
   maintaining (930) air in a needle (210) after the mixture expands and prior to using the device for an injection, such that air in the needle and the mixture are ready to be injected (940) into the eye.

2. The method of claim 1 wherein the air gap is calculated based on thermal expansion characteristics of the mixture.

3. The method of claim 1 wherein the air gap is located such that substantially no air is entrapped between the mixture and a plunger (415) on which the mixture sits.

4. The method of claim 1 wherein the mixture provided is a mixture of a lipophilic compound and a pharmaceutical.

5. The method of claim 1 wherein bringing the dispensing chamber housing (425) and mixture to a temperature range at which the mixture expands and is in a more liquid state further comprises bringing the mixture to a temperature range at which the mixture expands to substantially fill the dispensing chamber (405).

6. The method of claim 1 wherein the injection device has a needle (210) equal to or smaller than 25 gauge.

7. The method of claim 1 in which the mixture is a phase transition compound / drug mixture.

8. The method of claim 1 wherein heating the dispensing chamber housing (425) and mixture to a temperature range at which the mixture expands and is in a liquid state further comprises heating the dispensing chamber housing and mixture to about 75 degrees Celsius.

**Patentansprüche**

1. Verfahren zum Vorbereiten eines Injektionsgeräts (250) zum Injizieren eines Gemischs in ein Auge, mit:

Bereitstellen (910) des Gemischs in einer Dosierkammer (405), wobei sich zwischen dem Gemisch und einer Innenoberfläche eines Dosierkammergehäuses (425) ein Luftspalt befindet, wenn das Gemisch und das Dosierkammergehäuse etwa Raumtemperatur haben;
Bringen (920) das Dosierkammergehäuse und das Gemisch auf einen von etwa Raumtemperatur verschiedenen Temperaturbereich, bei dem sich das Gemisch ausdehnt und in einem mehr flüssigen Zustand ist;
Halten (930) von Luft in einer Nadel (210) nach der Ausdehnung des Gemischs und vor Verwenden des Geräts für eine Injektion, so dass die Luft in der Nadel und das Gemisch bereit sind, in das Auge injiziert (940) zu werden.

2. Verfahren nach Anspruch 1, bei dem der Luftspalt auf Basis der Wärmedehnungseigenschaften des Gemischs berechnet wird.

3. Verfahren nach Anspruch 1, bei dem der Luftspalt so angeordnet ist, dass im Wesentlichen keine Luft zwischen dem Gemisch und einem Kolben (415) eingeschlossen wird, auf dem das Gemisch ruht.

4. Verfahren nach Anspruch 1, bei dem das bereitgestellte Gemisch ein Gemisch aus einer lipophilen Verbindung und einem Pharmazeutikum ist.

5. Verfahren nach Anspruch 1, bei dem das Bringen des Dosierkammergehäuses (425) und des Gemischs auf einen Temperaturbereich, in dem sich das Gemisch ausdehnt und in einem mehr flüssigen Zustand ist, ferner das Bringen des Gemischs auf einen Temperaturbereich aufweist, in dem sich das Gemisch so ausdehnt, dass es die Dosierkammer (405) im Wesentlichen füllt.

6. Verfahren nach Anspruch 1, bei dem das Injektionsgerät eine Nadel (210) mit der Größe gleich oder kleiner 25 hat.

7. Verfahren nach Anspruch 1, bei dem das Gemisch ein Gemisch aus einer Verbindung mit Phasenübergang / Droge ist.

8. Verfahren nach Anspruch 1, bei dem das Erwärmen des Dosierkammergehäuses (425) und des Gemischs auf einen Temperaturbereich, in dem sich das Gemisch ausdehnt und in einem flüssigen Zustand ist, ferner das Erwärmen des Dosierkammergehäuses und des Gemischs auf ca. 75°C aufweist.

**Revendications**

1. Procédé de préparation d'un dispositif d'injection (250) pour injecter un mélange dans un oeil, comprenant les étapes consistant à :

fournir (910) le mélange dans une chambre de distribution (405) avec un intervalle d'air situé entre le mélange et une surface intérieure d'un logement de chambre de distribution (425), lorsque le mélange et le logement de chambre de distribution sont proches de la température ambiante ;
amener (920) le logement de chambre de distribution et le mélange à une plage de températures, autre qu'une température proche de la température ambiante, à laquelle le mélange se dilate et se trouve dans un état plus liquide ;
maintenir (930) de l'air dans une aiguille (210) après que le mélange soit dilaté et avant d'utiliser le dispositif en vue d'une injection, de sorte que l'air dans l'aiguille et le mélange soient prêts à être injectés (940) dans l'oeil.

2. Procédé selon la revendication 1, dans lequel l'intervalle d'air est calculé sur la base de caractéristiques de dilatation thermique du mélange.

3. Procédé selon la revendication 1, dans lequel l'intervalle d'air est situé de sorte que sensiblement pas d'air ne soit piégé entre le mélange et un piston (415) sur lequel le mélange se trouve.

4. Procédé selon la revendication 1, dans lequel le mélange fourni est un mélange d'un composé lipophile et d'un médicament.

5. Procédé selon la revendication 1, dans lequel l'opération consistant à amener le logement de chambre de distribution (425) et le mélange à une plage de températures à laquelle le mélange se dilate et est dans un état plus liquide comprend en outre l'opération consistant à amener le mélange à une plage de températures à laquelle le mélange se dilate pour sensiblement remplir la chambre de distribution (405).

6. Procédé selon la revendication 1, dans lequel le dispositif d'injection (250) comporte une aiguille (210) inférieure ou égale à 25 calibres.

7. Procédé selon la revendication 1, dans lequel le mélange est un mélange composé/médicament à transition de phase.

8. Procédé selon la revendication 1, dans lequel le chauffage du logement de chambre de distribution (425) et du mélange à une plage de températures à laquelle le mélange se dilate et est dans un état liquide comprend en outre le chauffage du logement de chambre de distribution et du mélange à environ 75 degrés Celsius.

105

110

115

120

125

130

Fig. 1 (Prior Art)

205

210

215

275

260

250

265

255

270

**Fig. 2**

340

308

330

250

320

**Fig. 3**

**Fig. 4**

**Fig. 5**

605     210

610

425

415

**Fig. 6A**

210

615

610

425

415

**Fig. 6B**

210

610

405

425

415

**Fig. 6C**

210

70° C

60° C

50° C

40° C

710

37° C

**Fig. 7**

210    807

**Fig. 8A**

817

210

**Fig. 8B**

210

827

**Fig. 8C**

Provide a substance in a dispensing chamber with an air gap between the mixture and the interior surface of the dispensing chamber housing at room temperature    910

Bring the mixture and the dispensing chamber housing to a temperature range at which the mixture expands and is in a more liquid state    920

Maintain air in the needle after the mixture expands and prior to injection    930

Drive the plunger at a rate to deposit the air in the needle and the mixture in the form of a shape into the eye    940

## Fig. 9

Provide a substance in a dispensing chamber with an air gap between the substance and the interior surface of the dispensing chamber housing at room temperature

1010

Bring the substance and the dispensing chamber housing to a temperature range at which the substance expands and is in a more liquid state

1020

Maintain air in the needle after the substance expands and prior to injection

1030

Select the drug release rate

1040

Drive the plunger at a rate so as to form a shape that results in the selected drug release rate

1050

**Fig. 10**

**EP 2 187 850 B1**

### Patent documents cited in the description

- US 6290690 B
- WO 2007024369 A
- US 69599007 A